# EUROPEAN PATENT APPLICATION

(11) **EP 4 484 557 A1**
(43) Date of publication of application: **01.01.2025**
(21) Application number: 23771040.5
(22) Date of filing: 10.03.2023
(51) Int. Cl.: C12N 9/00, C12N 15/77, C12N 1/20, C12P 13/10

(54) **CARBAMOYL-PHOSPHATE SYNTHETASE LARGE SUBUNIT VARIANT AND L-ORNITHINE PRODUCTION METHOD USING SAME**

(30) Priority: 18.03.2022 KR 20220034099
(71) Applicant: CJ CheilJedang Corporation, Seoul 04560 (KR)
(72) Inventor: PARK, Jinsub, Seoul 04560 (KR); BYUN, Hyo Jeong, Seoul 04560 (KR); HAN, Seunghee, Seoul 04560 (KR); LEE, Ji Yeon, Seoul 04560 (KR)
(74) Representative: Meissner Bolte Partnerschaft mbB
(86) International application number: PCT/KR2023/003303
(87) International publication number: WO 2023/177161

(57) **Abstract**

Provided are a carbamoyl phosphate synthetase large subunit variant, a polynucleotide encoding the variant, a microorganism comprising the variant or polynucleotide, and a method of producing L-ornithine using the same.

## Description

### [Technical Field]

The present disclosure relates to a carbamoyl phosphate synthetase large subunit variant, and a method of producing L-ornithine using the same.

### [Background Art]

In a process of producing L-amino acids using microorganisms, ATP is an important substance that is used as a cofactor for biosynthetic enzymes or in cell growth and various metabolic processes for active maintenance.

Carbamoyl phosphate is an intermediate metabolite used in the pyrimidine biosynthetic pathway and the arginine biosynthetic pathway. Carbamoyl phosphate synthetase is an enzyme that converts bicarbonate to carbamoyl phosphate, and utilizes two molecules of ATP and one molecule of glutamine as a nitrogen donor when producing carbamoyl phosphate. Glutamine is produced by glutamine synthetase from glutamic acid as a substrate, and in this process, one molecule of ATP is consumed.

In this regard, it has been reported that when a variant carbamoyl phosphate synthetase that desensitizes feedback inhibition and maintains high activity is introduced into E. *coli,* the production of L-arginine, citrulline, and pyrimidine derivatives increases (Patent Document 1).

However, it has been reported that excessive intracellular formation of carbamoyl phosphate causes cytotoxicity (Non-Patent Document 1). Carbamoyl phosphate is a very unstable substance, and cyanate, which is naturally converted from carbamoyl phosphate, may cause cytotoxicity.

Accordingly, if the activity of carbamoyl phosphate synthetase in microorganisms is controlled to maintain an appropriate intracellular level of carbamoyl phosphate, to prevent unnecessary waste of ATP, and to control energy balance, it is possible to improve L-amino acid production.

### [Prior Art Documents]

### [Patent Documents]

(Patent Document 1) JP 2003-093083 A

### [Non-Patent Documents]

(Non-Patent Document 1) Charlier et al., Amino Acids. 2018; 50(12): 1647-1661.

### [Disclosure]

### [Technical Problem]

The present inventors have developed a novel carbamoyl phosphate synthetase large subunit variant that increases L-ornithine production, a polynucleotide encoding the variant, a microorganism of the genus *Corynebacterium* comprising the variant or the polynucleotide, and a method of producing L-ornithine using the microorganism, thereby completing the present disclosure.

### [Technical Solution]

An object of the present disclosure is to provide a carbamoyl phosphate synthetase large subunit (CarB) variant derived from the genus *Corynebacterium,* the variant comprising a substitution of an amino acid corresponding to position 799 with isoleucine, leucine, or valine; a substitution of an amino acid corresponding to position 918 with leucine or valine; a substitution of an amino acid corresponding to position 1079 with isoleucine, leucine, or valine in an amino acid sequence of SEQ ID NO: 1; or a combination thereof.

Another object of the present disclosure is to provide a polynucleotide encoding the variant of the present disclosure.

Still another object of the present disclosure is to provide a microorganism of the genus *Corynebacterium* comprising the variant of the present disclosure; or a polynucleotide encoding the variant.

Still another object of the present disclosure is to provide a method of producing L-ornithine, the method comprising the step of culturing, in a medium, a microorganism of the genus *Corynebacterium* comprising the variant of the present disclosure; or a polynucleotide encoding the variant.

Still another object of the present disclosure is to provide a composition for producing L-ornithine, the composition comprising the variant of the present disclosure, a polynucleotide encoding the variant; a vector comprising the polynucleotide; a microorganism comprising the variant, the polynucleotide encoding the variant, or the vector comprising the polynucleotide; a culture of the microorganism, or a combination of two or more thereof.

Still another object of the present disclosure is to provide use of the microorganism of the present disclosure in producing L-ornithine.

Still another object of the present disclosure is to provide use of a carbamoyl phosphate synthetase large subunit (CarB) variant derived from the genus *Corynebacterium* in producing L-ornithine, wherein the variant comprises a substitution of an amino acid corresponding to position 799 with isoleucine, leucine, or valine; a substitution of an amino acid corresponding to position 918 with leucine or valine; a substitution of an amino acid corresponding to position 1079 with isoleucine, leucine, or valine in an amino acid sequence of SEQ ID NO: 1; or a combination thereof.

### [Advantageous Effects]

A microorganism into which a variant of the present disclosure is introduced may have increased L-ornithine-producing ability, as compared to existing unmodified microorganisms.

### [Detailed Description of Preferred Embodiments]

The present disclosure will be described in detail as follows. Meanwhile, each description and embodiment disclosed in this disclosure may also be applied to other descriptions and embodiments. That is, all combinations of various elements disclosed in this disclosure fall within the scope of the present disclosure. Further, the scope of the present disclosure is not limited by the specific description described below. Further, a number of papers and patent documents are referenced and cited throughout this specification. The disclosures of the cited papers and patent documents are incorporated herein by reference in their entirety to further clarify the level and scope of the subject matter to which the present disclosure pertains.

An aspect of the present disclosure provides a carbamoyl phosphate synthetase large subunit (CarB) variant derived from the genus *Corynebacterium,* the variant comprising a substitution of an amino acid corresponding to position 799 with a different amino acid; a substitution of an amino acid corresponding to position 918 with a different amino acid; a substitution of an amino acid corresponding to position 1079 with a different amino acid in an amino acid sequence of SEQ ID NO: 1; or a combination thereof.

The variant of the present disclosure may be a variant in which any one or more of the amino acids corresponding to position 799, 918, or 1079 in the amino acid sequence of SEQ ID NO: 1 are substituted with an amino acid different from the amino acid before substitution. Alternatively, the variant may be a variant having uncharged amino acids, in which an amino acid different from the amino acid before substitution is substituted, but is not limited thereto.

Specifically, the variant may be a variant in which i) the amino acid corresponding to position 799 is substituted with any one or more amino acids selected from the group consisting of arginine, lysine, histidine, aspartic acid, glycine, alanine, valine, leucine, isoleucine, methionine, phenylalanine, tryptophan, proline, serine, threonine, cysteine, tyrosine, asparagine, and glutamine, ii) the amino acid corresponding to position 918 is substituted with any one or more amino acids selected from the group consisting of arginine, lysine, histidine, aspartic acid, glycine, alanine, valine, leucine, isoleucine, methionine, phenylalanine, tryptophan, proline, serine, threonine, cysteine, tyrosine, asparagine, and glutamine, or iii) the amino acid corresponding to position 1079 is substituted with any one or more amino acids selected from the group consisting of arginine, lysine, histidine, glutamic acid, aspartic acid, glycine, alanine, valine, leucine, isoleucine, methionine, phenylalanine, tryptophan, proline, serine, cysteine, tyrosine, asparagine, and glutamine in the amino acid sequence of SEQ ID NO: 1, but is not limited thereto.

More specifically, the variant may be a variant in which i) the amino acid corresponding to position 799 is substituted with any one or more amino acids selected from the group consisting of alanine, isoleucine, leucine, and valine, ii) the amino acid corresponding to position 918 is substituted with any one or more amino acids selected from the group consisting of alanine, isoleucine, leucine, and valine, or iii) the amino acid corresponding to position 1079 is substituted with any one or more amino acids selected from the group consisting of alanine, isoleucine, leucine, and valine in the amino acid sequence of SEQ ID NO: 1, but is not limited thereto.

Much more specifically, the variant may have, comprise, or consist of an amino acid sequence represented by SEQ ID NO: 3 or SEQ ID NO: 5 or SEQ ID NO: 7 or SEQ ID NO: 9 or SEQ ID NO: 11 or SEQ ID NO: 13 or SEQ ID NO: 15 or SEQ ID NO: 17 or SEQ ID NO: 19 or SEQ ID NO: 21 or SEQ ID NO: 23 or SEQ ID NO: 25, or may essentially consist of the amino acid sequence.

The variant of the present disclosure may be a variant in which, based on the amino acid of SEQ ID NO: 1, the amino acid corresponding to position 799 is any one or more amino acids selected from the group consisting of alanine, isoleucine, leucine, and valine, the amino acid corresponding to position 918 is any one or more amino acids selected from the group consisting of alanine, isoleucine, leucine, and valine, and/or the amino acid corresponding to position 1079 is any one or more amino acids selected from the group consisting of alanine, isoleucine, leucine, and valine in the amino acid sequence represented by SEQ ID NO: 3 or SEQ ID NO: 5 or SEQ ID NO: 7 or SEQ ID NO: 9 or SEQ ID NO: 11 or SEQ ID NO: 13 or SEQ ID NO: 15 or SEQ ID NO: 17 or SEQ ID NO: 19 or SEQ ID NO: 21 or SEQ ID NO: 23 or SEQ ID NO: 25, and may comprise an amino acid sequence having at least 70%, 75%, 80%, 85%, 90%, 95%, 96%, 97%, 98%, 99%, 99.5%, 99.7%, or 99.9% or more homology or identity to the amino acid sequence represented by SEQ ID NO: 1. Further, it is apparent that any variant having an amino acid sequence with deletion, modification, substitution, conservative substitution, or addition in part of the sequence may also fall within the scope of the present disclosure, as long as the amino acid sequence has such a homology or identity and exhibits efficacy corresponding to that of the variant of the present disclosure.

Examples thereof comprise those having sequence addition or deletion that do not alter the function of the variant of the present disclosure at the N-terminus, C-terminus of the amino acid sequence, and/or inside the amino acid sequence, a naturally occurring mutation, a silent mutation, or a conservative substitution.

The "conservative substitution" means the substitution of one amino acid with another amino acid having similar structural and/or chemical properties. Such an amino acid substitution may generally occur based on similarity in the polarity, charge, solubility, hydrophobicity, hydrophilicity, and/or amphipathic nature of residues. Usually, conservative substitution may hardly affect or not affect the activity of the proteins or polypeptides.

For example, among amino acids having electrically charged side chains (electrically charged amino acids), positively charged (basic) amino acids comprise arginine, lysine, and histidine, negatively charged (acidic) amino acids comprise glutamic acid and aspartic acid; amino acids having uncharged side chains comprise glycine, alanine, valine, leucine, isoleucine, methionine, phenylalanine, tryptophan, proline, serine, threonine, cysteine, tyrosine, asparagine, and glutamine.

As used herein, the term "variant" refers to a polypeptide in which one or more amino acids are conservatively substituted and/or modified so as to be different from the amino acid sequence of the variant before modification, while retaining functions or properties thereof. Such a variant may generally be identified by modifying one or more amino acids in the amino acid sequence of the polypeptide and evaluating the properties of the modified polypeptide. In other words, the ability of the variant may be increased, unchanged, or decreased, as compared to the polypeptide before modification. Further, some variants may comprise variants in which one or more portions, such as an N-terminal leader sequence or transmembrane domain, have been removed. Other variants may comprise variants in which a portion has been removed from the N- and/or C-terminus of a mature protein. The term "variant" may also be used to refer to a modification, modified polypeptide, modified protein, mutant, mutein, divergent, etc., and any term is not limited, as long as it is used in the sense of being mutated. With respect to the objects of the present disclosure, the variant may be a polypeptide comprising an amino acid sequence represented by SEQ ID NO: 3 or SEQ ID NO: 5 or SEQ ID NO: 7 or SEQ ID NO: 9 or SEQ ID NO: 11 or SEQ ID NO: 13 or SEQ ID NO: 15 or SEQ ID NO: 17 or SEQ ID NO: 19 or SEQ ID NO: 21 or SEQ ID NO: 23 or SEQ ID NO: 25, in which glutamic acid which is the amino acid corresponding to position 799 is substituted with any one or more amino acids selected from the group consisting of alanine, isoleucine, leucine, and valine, glutamic acid which is the amino acid corresponding to position 918 is substituted with any one or more amino acids selected from the group consisting of alanine, isoleucine, leucine, and valine, and/or threonine which is the amino acid corresponding to position 1079 is substituted with any one or more amino acids selected from the group consisting of alanine, isoleucine, leucine, and valine in the amino acid sequence of SEQ ID NO: 1.

The variant may also comprise deletion or addition of amino acids that have minimal influence on the properties and secondary structure of the polypeptide. For example, a polypeptide may be conjugated to a signal (or leader) sequence at the N-terminus of the variant, which co-translationally or post-translationally directs the transfer of the protein. The variant may also be conjugated to other sequence or a linker for identification, purification, or synthesis.

As used herein, the term 'homology' or 'identity' means the degree of similarity between two given amino acid sequences or nucleotide sequences and may be expressed as a percentage. The terms 'homology' and 'identity' may often be used interchangeably.

The sequence homology or identity of a conserved polynucleotide or polypeptide is determined by standard alignment algorithms, and the default gap penalty established by the used program may be used together. Substantially, homologous or identical sequences are generally capable of being hybridized with the entirety or a part of the sequence under moderately or highly stringent conditions. It is apparent that hybridization also comprises hybridization with a polynucleotide comprising a general codon or a codon in consideration of codon degeneracy.

Whether any two polynucleotide or polypeptide sequences have homology, similarity, or identity may be determined using known computer algorithms such as the "FASTA" program, for example, using default parameters as in Pearson et al (1988)[Proc. Natl. Acad. Sci. USA 85]: 2444. Alternatively, the homology, similarity, or identity may be determined using the Needleman-Wunsch algorithm (Needleman and Wunsch, 1970, J. Mol. Biol. 48: 443-453) as performed in the Needleman program of the EMBOSS package (EMBOSS: The European Molecular Biology Open Software Suite, Rice et al., 2000, Trends Genet. 16: 276-277) (version 5.0.0 or later) (comprising GCG program package (Devereux, J., et al, Nucleic Acids Research 12: 387 (1984)), BLASTP, BLASTN, FASTA (Atschul, [S.] [F.,] [ETAL, J MOLEC BIOL 215]: 403 (1990); Guide to Huge Computers, Martin J. Bishop, [ED.,] Academic Press, San Diego,1994, and [CARILLO ET AL/.] (1988) SIAM J Applied Math 48: 1073). For example, BLAST of the National Center for Biotechnology Information, or ClustalW may be used to determine the homology, similarity, or identity.

The homology, similarity, or identity of polynucleotides or polypeptides may be determined by comparing sequence information using, for example, a GAP computer program such as Needleman et al. (1970), J Mol Biol. 48:443 as announced in, for example, Smith and Waterman, Adv. Appl. Math (1981) 2:482. In summary, the GAP program may be defined as the value acquired by dividing the number of similarly aligned symbols (namely, nucleotides or amino acids) by the total number of symbols in the shorter of two sequences. The default parameters for the GAP program may comprise (1) a binary comparison matrix (comprising values of 1 for identity and 0 for non-identity) and a weighted comparison matrix of Gribskov et al(1986) Nucl. Acids Res. 14: 6745 (or EDNAFULL (EMBOSS version of NCBI NUC4.4) substitution matrix), as disclosed in Schwartz and Dayhoff, eds., Atlas Of Protein Sequence And Structure, National Biomedical Research Foundation, pp. 353-358 (1979); (2) a penalty of 3.0 for each gap and an additional 0.10 penalty for each symbol in each gap (or gap opening penalty of 10, gap extension penalty of 0.5); and (3) no penalty for end gaps.

In one embodiment of the present disclosure, the variant of the present disclosure may have increased L-ornithine-producing ability. Specifically, the variant of the present disclosure may have the activity to increase L-ornithine-producing ability, as compared to a wild-type polypeptide having carbamoyl phosphate synthetase activity.

As used herein, the term "carbamoyl phosphate synthetase" refers to an enzyme that converts bicarbonate to carbamoyl phosphate.

The carbamoyl phosphate synthetase may consist of a carbamoyl phosphate synthetase small subunit (CarA) and a carbamoyl phosphate synthetase large subunit (CarB), and an amino acid sequence of CarA or CarB may be obtained from NCBI's Genbank which is a known database, etc.

For example, CarB of the present disclosure may be derived from a microorganism. The microorganism may be derived from, specifically, a microorganism of the genus Corynebacterium, more specifically, *Corynebacterium glutamicum, Corynebacterium deserti, Corynebacterium crudilactis, Corynebacterium efficiens, Corynebacterium callunae,* etc., but is not limited thereto. With respect to the objects of the present disclosure, the present disclosure may provide a variant of CarB derived from *Corynebacterium glutamicum.*

For another example, the amino acid of CarB of the present disclosure may be ANU33812.1 derived from *Corynebacterium glutamicum* ATCC 13869, but it is obvious that proteins with CarB activity, derived from various origins, are comprised.

With respect to the objects of the present disclosure, the variant of the present disclosure may be the carbamoyl phosphate synthetase large subunit variant, i.e., CarB variant.

CarB may have, comprise, or consist of the amino acid sequence represented by SEQ ID NO: 1, or may essentially consist of the amino acid sequence.

The amino acid sequence of SEQ ID NO: 1 may be obtained from the NIH Genbank, which is a known database, etc. In the present disclosure, the amino acid sequence of SEQ ID NO: 1 may comprise an amino acid sequence having at least 70%, 75%, 80%, 85%, 90%, 95%, 96%, 97%, 98%, 99%, 99.5%, 99.7%, or 99.9% or more homology or identity to the amino acid sequence represented by SEQ ID NO: 1. Further, it is apparent that any protein having an amino acid sequence with deletion, modification, substitution, conservative substitution or addition in part of the sequence may also fall within the scope of the present disclosure, as long as the amino acid sequence has such a homology or identity and exhibits efficacy corresponding to that of the protein comprising the amino acid sequence of SEQ ID NO: 1.

Examples thereof comprise those having sequence addition or deletion that do not alter the function of the protein of the present disclosure at the N-terminus, C-terminus of the amino acid sequence, and/or inside the amino acid sequence, a naturally occurring mutation, a silent mutation, or a conservative substitution.

As used herein, the term "corresponding to" refers to amino acid residues at positions listed in the polypeptide, or amino acid residues that are similar, identical, or homologous to those listed in the polypeptide. Identifying the amino acid at the corresponding position may involve determining a specific amino acid in a sequence that corresponds to a specific sequence. As used herein, "corresponding region" generally refers to a similar or corresponding position in a related protein or a reference protein.

For example, an arbitrary amino acid sequence is aligned with SEQ ID NO: 1, and based on this, each amino acid residue of the amino acid sequence may be numbered with reference to the amino acid residue of SEQ ID NO: 1 and the numerical position of the corresponding amino acid residue. For example, a sequence alignment algorithm as described in the present disclosure may determine the position of an amino acid or a position at which modification such as substitution, insertion, or deletion occurs through comparison with that in a query sequence (also referred to as a "reference sequence").

For such alignments, for example, the Needleman-Wunsch algorithm (Needleman and Wunsch, 1970, J. Mol. Biol. 48: 443-453), the Needleman program of the EMBOSS package (EMBOSS: The European Molecular Biology Open Software Suite, Rice et al., 2000, Trends Genet. 16:276-277), and the like may be used, but are not limited thereto, and a sequence alignment program, a pairwise sequence comparison algorithm, and the like known in the art may be appropriately used.

Another aspect of the present disclosure provides a polynucleotide encoding the variant of the present disclosure.

As used herein, the term "polynucleotide", which is a polymer of nucleotides, in which nucleotide monomers are linked in a long chain shape by covalent bonds, refers to a DNA or RNA strand having a predetermined or longer length, more specifically, a polynucleotide fragment encoding the variant.

The polynucleotide encoding the variant of the present disclosure may comprise a nucleotide sequence encoding an amino acid sequence represented by SEQ ID NO: 3 or SEQ ID NO: 5 or SEQ ID NO: 7 or SEQ ID NO: 9 or SEQ ID NO: 11 or SEQ ID NO: 13 or SEQ ID NO: 15 or SEQ ID NO: 17 or SEQ ID NO: 19 or SEQ ID NO: 21 or SEQ ID NO: 23 or SEQ ID NO: 25. In one embodiment of the present disclosure, the polynucleotide of the present disclosure may have or comprise a sequence of SEQ ID NO: 4 or SEQ ID NO: 6 or SEQ ID NO: 8 or SEQ ID NO: 10 or SEQ ID NO: 12 or SEQ ID NO: 14 or SEQ ID NO: 16 or SEQ ID NO: 18 or SEQ ID NO: 20 or SEQ ID NO: 22 or SEQ ID NO: 24 or SEQ ID NO: 26. Further, the polynucleotide of the present disclosure may consist of or essentially consist of the sequence of SEQ ID NO: 4 or SEQ ID NO: 6 or SEQ ID NO: 8 or SEQ ID NO: 10 or SEQ ID NO: 12 or SEQ ID NO: 14 or SEQ ID NO: 16 or SEQ ID NO: 18 or SEQ ID NO: 20 or SEQ ID NO: 22 or SEQ ID NO: 24 or SEQ ID NO: 26.

In the polynucleotide of the present disclosure, various modifications may be made in the coding region as long as the amino acid sequence of the variant of the present disclosure is not changed in consideration of codon degeneracy or codons preferred in organisms that are intended to express the variant of the present disclosure. Specifically, the polynucleotide of the present disclosure may have or comprise a nucleotide sequence having 70% or more, 75% or more, 80% or more, 85% or more, 90% or more, 95% or more, 96% or more, 97% or more, 98% or more, and less than 100% homology or identity to the sequence of SEQ ID NO: 4 or SEQ ID NO: 6 or SEQ ID NO: 8 or SEQ ID NO: 10 or SEQ ID NO: 12 or SEQ ID NO: 14 or SEQ ID NO: 16 or SEQ ID NO: 18 or SEQ ID NO: 20 or SEQ ID NO: 22 or SEQ ID NO: 24 or SEQ ID NO: 26, or may consist of or essentially consist of a nucleotide sequence having 70% or more, 75% or more, 80% or more, 85% or more, 90% or more, 95% or more, 96% or more, 97% or more, 98% or more, and less than 100% homology or identity to the sequence of SEQ ID NO: 4 or SEQ ID NO: 6 or SEQ ID NO: 8 or SEQ ID NO: 10 or SEQ ID NO: 12 or SEQ ID NO: 14 or SEQ ID NO: 16 or SEQ ID NO: 18 or SEQ ID NO: 20 or SEQ ID NO: 22 or SEQ ID NO: 24 or SEQ ID NO: 26, but is not limited thereto. In this regard, in the sequence having such homology or identity, a codon encoding the amino acid corresponding to position 799 of SEQ ID NO: 1 may be one of the codons encoding any one or more amino acids selected from the group consisting of alanine, isoleucine, leucine, and valine; a codon encoding the amino acid corresponding to position 918 of SEQ ID NO: 1 may be one of the codons encoding any one or more amino acids selected from the group consisting of alanine, isoleucine, leucine, and valine; and a codon encoding the amino acid corresponding to position 1079 of SEQ ID NO: 1 may be one of the codons encoding any one or more amino acids selected from the group consisting of alanine, isoleucine, leucine, and valine.

Further, the polynucleotide of the present disclosure may comprise a probe that may be prepared from a known gene sequence, for example, any sequence without limitation as long as it is a sequence that is able to hybridize with a complementary sequence to the entirety or a part of the polynucleotide sequence of the present disclosure under stringent conditions. The term "stringent conditions" refers to conditions that enable specific hybridization between polynucleotides. These conditions are specifically described in documents (see J. Sambrook et al., Molecular Cloning, A Laboratory Manual, 2nd Edition, Cold Spring Harbor Laboratory Press, Cold Spring Harbor, New York, 1989; F.M. Ausubel et al.,Current Protocols in Molecular Biology, John Wiley & Sons, Inc., New York, 9.50-9.51, 11.7-11.8). Examples thereof comprise conditions under which polynucleotides having higher homology or identity, namely, polynucleotides having 70% or more, 75% or more, 76% or more, 85% or more, 90% or more, 95% or more, 96% or more, 97% or more, 98% or more, or 99% or more homology or identity are hybridized with each other while polynucleotides having lower homology or identity are not hybridized with each other, or conditions under which washing is performed once, specifically twice to three times at a salt concentration and temperature equivalent to 60°C, 1×SSC, 0.1% SDS, specifically 60°C, 0.1×SSC, 0.1% SDS, more specifically 68°C, 0.1×SSC, 0.1% SDS, which are washing conditions for common Southern hybridization.

Hybridization requires that two nucleic acids have complementary sequences, although mismatches between bases are allowed depending on the stringency of hybridization. The term "complementary" is used to describe the relationship between nucleotide bases capable of being hybridized with each other. For example, with regard to DNA, adenine is complementary to thymine and cytosine is complementary to guanine. Hence, the polynucleotide of the present disclosure may also comprise substantially similar nucleic acid sequences as well as isolated nucleic acid fragments that are complementary to the entire sequence.

Specifically, a polynucleotide having homology or identity to the polynucleotide of the present disclosure may be detected using a hybridization condition comprising a hybridization step at a Tm value of 55°C and the above-described conditions. The Tm value may be 60°C, 63°C, or 65°C, but is not limited thereto, and may be appropriately adjusted by those skilled in the art according to the purpose.

The appropriate stringency to hybridize the polynucleotide depends on the length and degree of complementarity of the polynucleotide, and the variables are well known in the art (e.g., J. Sambrook et al., supra).

Still another aspect of the present disclosure provides a vector comprising the polynucleotide of the present disclosure. The vector may be an expression vector for expressing the polynucleotide in a host cell, but is not limited thereto.

The vector of the present disclosure may comprise a DNA construct comprising a nucleotide sequence of a polynucleotide encoding a desired polypeptide which is operably linked to a suitable expression regulatory region (or expression control sequence) so that the desired polypeptide may be expressed in a suitable host. The expression regulatory region may comprise a promoter capable of initiating transcription, any operator sequence for controlling transcription, a sequence encoding a suitable mRNA ribosome binding site, and a sequence controlling termination of transcription and translation. The vector may be transformed into a suitable host cell, and then replicated or function independently of the host genome, or may be integrated into the genome itself.

The vector used in the present disclosure is not particularly limited, but any vector known in the art may be used. Examples of commonly used vectors comprise natural or recombinant plasmids, cosmids, viruses, and bacteriophages. For example, pWE15, M13, MBL3, MBL4, IXII, ASHII, APII, t10, t11, Charon4A, Charon21A, etc. may be used as a phage vector or a cosmid vector, and pDZ system, pBR system, pUC system, pBluescript II system, pGEM system, pTZ system, pCL system, pET system, etc. may be used as a plasmid vector. Specifically, pDZ, pDC, pDCM2, pACYC177, pACYC184, pCL, pECCG117, pUC19, pBR322, pMW118, pCC1BAC vector, etc. may be used.

For example, a polynucleotide encoding a desired polypeptide may be inserted into a chromosome through a vector for intracellular chromosome insertion. Insertion of the polynucleotide into the chromosome may be performed by any method known in the art, for example, homologous recombination, but is not limited thereto. The vector may further comprise a selection marker for the confirmation of chromosome insertion. The selection marker is for selecting the cells transformed with vectors, i.e., for confirming the insertion of a desired nucleic acid molecule, and markers that confer selectable phenotypes such as drug resistance, auxotrophy, resistance to cytotoxic agents, or expression of surface polypeptides may be used. In an environment treated with a selective agent, only cells expressing the selection marker survive or exhibit other phenotypic traits, and thus transformed cells may be selected.

As used herein, the term "transformation" means that a vector comprising a polynucleotide encoding a target polypeptide is introduced into a host cell or a microorganism so that the polypeptide encoded by the polynucleotide may be expressed in the host cell. The transformed polynucleotide may be located regardless of the position, either by being inserted into the chromosome of the host cell or located outside the chromosome as long as it may be expressed in the host cell. Further, the polynucleotide comprises DNA and/or RNA encoding a desired polypeptide. The polynucleotide may be introduced in any form as long as it may be introduced into a host cell and expressed. For example, the polynucleotide may be introduced into a host cell in the form of an expression cassette, which is a gene construct comprising all elements required for autonomous expression. The expression cassette may usually comprise a promoter operably linked to the polynucleotide, a transcription termination signal, a ribosome binding site, and a translation termination signal. The expression cassette may be in the form of an expression vector capable of self-replicating. Further, the polynucleotide may be introduced into a host cell in its own form and operably linked to a sequence required for expression in the host cell, but is not limited thereto.

Further, the term "operably linked" means that the polynucleotide sequence is functionally linked to a promoter sequence that initiates and mediates transcription of the polynucleotide encoding the desired variant of the present disclosure.

Still another aspect of the present disclosure provides a microorganism comprising the variant of the present disclosure or a microorganism comprising the polynucleotide of the present disclosure.

As used herein, the term "microorganism (or strain)" comprises all wild-type microorganisms or naturally or artificially genetically modified microorganisms, and it may be a microorganism in which a specific mechanism is weakened or strengthened due to an insertion of a foreign gene or an activity enhancement or inactivation of an endogenous gene, and it may be a microorganism comprising a genetic modification for the production of a target polypeptide, protein, or product.

The strain of the present disclosure may be a strain comprising any one or more of the variant of the present disclosure, the polynucleotide of the present disclosure, and the vector comprising the polynucleotide of the present disclosure; a strain modified to express the variant of the present disclosure or the polynucleotide of the present disclosure; a strain (e.g., recombinant strain) expressing the variant of the present disclosure or the polynucleotide of the present disclosure; or a strain (e.g., recombinant strain) having the activity of the variant of the present disclosure, but is not limited thereto.

In one embodiment of the present disclosure, the strain of the present disclosure may be a strain having the L-ornithine-producing ability.

The microorganism of the present disclosure may be a microorganism having the improved L-ornithine-producing ability.

The strain of the present disclosure may be a microorganism naturally having carbamoyl phosphate synthetase or L-amino acid-producing ability or a microorganism prepared by introducing the variant of the present disclosure or the polynucleotide (or vector comprising the polynucleotide) encoding the same into a parent strain and/or by providing the L-ornithine-producing ability, but is not limited thereto.

For example, the strain of the present disclosure is a cell or microorganism that is transformed with the polynucleotide of the present disclosure or the vector comprising the polynucleotide encoding the variant of the present disclosure to express the variant of the present disclosure. With respect to the objects of the present disclosure, the strain of the present disclosure may comprise all microorganisms capable of producing L-ornithine by comprising the variant of the present disclosure. For example, the strain of the present disclosure may be a recombinant strain with increased L-ornithine-producing ability, in which the carbamoyl phosphate synthetase large subunit variant is expressed by introducing the polynucleotide encoding the variant of the present disclosure into a natural wild-type microorganism or a microorganism producing L-ornithine. The recombinant strain with increased L-ornithine-producing ability may be a microorganism in which L-ornithine-producing ability is increased, as compared to a natural wild-type microorganism or a carbamoyl phosphate synthetase-unmodified microorganism (e.g., a microorganism expressing the wild-type carbamoyl phosphate synthetase (SEQ ID NO: 1) or a microorganism not expressing the variant (SEQ ID NO: 3 or SEQ ID NO: 5 or SEQ ID NO: 7 or SEQ ID NO: 9 or SEQ ID NO: 11 or SEQ ID NO: 13 or SEQ ID NO: 15 or SEQ ID NO: 17 or SEQ ID NO: 19 or SEQ ID NO: 21 or SEQ ID NO: 23 or SEQ ID NO: 25) protein, but is not limited thereto. For example, the carbamoyl phosphate synthetase-unmodified microorganism, which is a target strain for comparing whether or not L-ornithine-producing ability increases, may be a wild-type *Corynebacterium glutamicum* ATCC 13869 strain, a *Corynebacterium glutamicum ATCC* 13869 strain with weakened ArgF protein activity, which is known as an L-ornithine producing strain, or a *Corynebacterium glutamicum* ATCC 13869 strain with weakened ArgR protein activity, but is not limited thereto.

For example, the recombinant strain having increased production ability may have increased L-ornithine-producing ability of about 1% or more, specifically, about 1% or more, about 2.5% or more, about 5% or more, about 6% or more, about 7% or more, about 8% or more, about 9% or more, about 10% or more, about 15% or more, about 16% or more, about 17% or more, about 18% or more, about 19% or more, or about 20% or more (the upper limit is not particularly limited, but may be, for example, about 200% or less, about 150% or less, about 100% or less, about 50% or less, about 40% or less, about 30% or less, about 20% or less, or about 15% or less), as compared to that of the parent strain before modification or the unmodified microorganism, but the increased amount is not limited thereto as long as the production ability has an increased amount of a + value, as compared to the production ability of the parent strain before modification or the unmodified microorganism. In another example, the recombinant strain having increased production ability may have increased L-ornithine-producing ability of about 1.01 times or more, about 1.02 times or more, about 1.03 times or more, about 1.05 times or more, about 1.06 times or more, about 1.07 times or more, about 1.08 times or more, about 1.09 times or more, about 1.10 times or more, about 1.15 times or more, about 1.16 times or more, about 1.17 times or more, about 1.18 times or more, about 1.19 times or more, or about 1.20 times or more (the upper limit is not particularly limited, but may be, for example, about 10 times or less, about 5 times or less, about 3 times or less, or about 2 times or less), as compared to that of the parent strain before modification or the unmodified microorganism, but is not limited thereto.

As used herein, the term "unmodified microorganism" does not exclude strains comprising mutation that may occur naturally in microorganisms, and may be a wild-type strain or a natural strain itself or may be a strain before the trait is changed by genetic variation due to natural or artificial factors. For example, the unmodified microorganism may refer to a strain in which the carbamoyl phosphate synthetase large subunit variant described herein is not introduced or has not yet been introduced. The "unmodified microorganism" may be used interchangeably with "strain before being modified", "microorganism before being modified", "unvaried strain", "unmodified strain", "unvaried microorganism", or "reference microorganism".

In another embodiment of the present disclosure, the microorganism of the present disclosure may be *Corynebacterium stationis, Corynebacterium glutamicum, Corynebacterium crudilactis, Corynebacterium deserti, Corynebacterium efficiens, Corynebacterium callunae, Corynebacterium singulare, Corynebacterium halotolerans, Corynebacterium striatum, Corynebacterium ammoniagenes, Corynebacterium pollutisoli, Corynebacterium imitans, Corynebacterium testudinoris,* or *Corynebacterium flavescens.*

In still another embodiment of the present disclosure, the recombinant microorganism of the present disclosure may be a microorganism in which L-amino acid-producing ability is enhanced by additionally enhancing the activity of a part of proteins in the L-amino acid biosynthetic pathway or by additionally weakening the activity of a part of proteins in the L-amino acid degradation pathway.

Specifically, the microorganism of the present disclosure may be a microorganism in which the activity of ornithine carbamoyltransferase subunit F (ArgF) is additionally weakened, or the argF gene encoding the same is additionally deleted, and/or the activity of arginine repressor (ArgR) is additionally weakened, or the argR gene encoding the same is additionally deleted.

An amino acid sequence of ArgF or ArgR may be obtained from NCBI's Genbank, which is a known database, etc. For example, the amino acid sequence of ArgF of the present disclosure may comprise ANU33618.1 (SEQ ID NO: 48) derived from *Corynebacterium glutamicum ATCC* 13869 or an amino acid sequence having 90% or more homology thereto, and the amino acid sequence of ArgR may comprise ANU33619.1 (SEQ ID NO: 53) derived from *Corynebacterium glutamicum* ATCC 13869 or an amino acid sequence having 90% or more homology thereto, but is not limited thereto, and it is obvious that proteins with ArgF or ArgR activity, derived from various origins, are comprised.

However, the weakening of ArgF and/or ArgR protein activity or the deletion of the argF and/or argR gene is only one example and is not limited thereto, and the microorganism of the present disclosure may be a microorganism in which the activities of the proteins in various known L-amino acid biosynthetic pathways are enhanced or the activities of the proteins in the degradation pathway are weakened.

As used herein, the term "weakening" of the activity of the polypeptide has a concept encompassing all of the weakening of activity or the absence of activity, as compared to the endogenous activity. The weakening may be used interchangeably with terms such as inactivation, deficiency, down-regulation, decrease, reduction, attenuation, etc.

The weakening may also comprise a case where the activity of the polypeptide itself is weakened or eliminated due to a variation of the polynucleotide encoding the polypeptide, etc., as compared to the activity of the polypeptide originally possessed by the microorganism, a case where the overall polypeptide activity level and/or concentration (expression level) in the cell is low due to inhibition of the expression of the gene of the polynucleotide encoding the polypeptide or due to inhibition of translation into the polypeptide, as compared to that of the natural strain, a case where the polynucleotide is not expressed at all, and/or a case where the polypeptide activity is absent even when the polynucleotide is expressed. The "endogenous activity" means the activity of a specific polypeptide originally possessed by a parent strain before the trait is changed or a wild-type or unmodified microorganism, when the trait is changed by genetic variation due to natural or artificial factors. This may be used interchangeably with "activity before modification". The "inactivation, deficiency, decrease, down-regulation, reduction, or attenuation" of the activity of a polypeptide compared to the endogenous activity thereof means that the activity of the polypeptide is lowered, compared to the activity of a specific polypeptide originally possessed by a parent strain before the trait is changed or an unmodified microorganism.

Such weakening of the activity of the polypeptide may be performed by any method known in the art, but is not limited thereto, and may be achieved by applying various methods well known in the art (e.g., Nakashima N et al., Bacterial cellular engineering by genome editing and gene silencing. Int J Mol Sci. 2014;15(2):2773-2793, Sambrook et al. Molecular Cloning 2012, etc.).

Specifically, the weakening of the activity of the polypeptide of the present disclosure may be achieved by:
1) deletion of the entirety or a part of the gene encoding the polypeptide;
2) modification of an expression control region (or expression control sequence) to reduce the expression of a gene encoding the polypeptide;
3) modification of the amino acid sequence constituting the polypeptide to eliminate or weaken the activity of the polypeptide (e.g., deletion/substitution/addition of one or more amino acids in the amino acid sequence);
4) modification of the gene sequence encoding the polypeptide to eliminate or weaken the activity of the polypeptide (e.g., deletion/substitution/addition of one or more nucleotides in the nucleotide sequence of the polypeptide gene to encode the polypeptide which is modified to eliminate or weaken the activity of the polypeptide);
5) modification of a nucleotide sequence encoding an initiation codon or 5'-UTR region of the gene transcript encoding the polypeptide;
6) introduction of an antisense oligonucleotide (e.g., antisense RNA) complementarily binding to the gene transcript encoding the polypeptide;
7) addition of a sequence complementary to the Shine-Dalgarno sequence of the gene encoding the polypeptide to the upstream of the Shine-Dalgarno sequence in order to form a secondary structure that makes the attachment of ribosomes impossible;
8) addition of a promoter, which is to be reverse-transcribed, to the 3' end of the open reading frame (ORF) of the gene sequence encoding the polypeptide (reverse transcription engineering, RTE); or
9) combination of two or more selected from 1) to 8), but is not particularly limited thereto.

For example,
1) The deletion of a part or the entirety of the gene encoding the polypeptide may be elimination of the entirety of the polynucleotide encoding an endogenous target polypeptide in the chromosome, replacement with a polynucleotide having deletion of some nucleotides, or replacement with a marker gene.
2) The modification of an expression control region (or expression control sequence) may be the mutation on the expression control region (or expression control sequence) through deletion, insertion, non-conservative or conservative substitution, or a combination thereof, or the replacement with a sequence having weaker activity. The expression control region comprises a promoter, an operator sequence, a sequence for encoding a ribosomal binding site, and a sequence for controlling the termination of transcription and translation, but is not limited thereto.
Further, 3) and 4) the modification of the amino acid sequence or the polynucleotide sequence may be mutation on the sequence through deletion, insertion, non-conservative or conservative substitution, or a combination thereof in the amino acid sequence of the polypeptide or the polynucleotide sequence encoding the polypeptide, or replacement with an amino acid sequence or polynucleotide sequence improved to have weaker activity, or an amino acid sequence or polynucleotide sequence improved to have no activity, so as to weaken activity of the polypeptide, but is not limited thereto. For example, the expression of the gene may be inhibited or attenuated by introducing mutation into the polynucleotide sequence to form a stop codon, but is not limited thereto.
5) The modification of a nucleotide sequence encoding an initiation codon or 5'-UTR region of the gene transcript encoding the polypeptide may be, for example, the substitution with a nucleotide sequence encoding, rather than an endogenous initiation codon, another initiation codon having a lower expression rate of the polypeptide, but is not limited thereto.
6) The introduction of an antisense oligonucleotide (e.g., antisense RNA) complementarily binding to the gene transcript encoding the polypeptide may be referred to, for example, a literature [Weintraub, H. et al., Antisense-RNA as a molecular tool for genetic analysis, Reviews - Trends in Genetics, Vol. 1(1) 1986].
7) The addition of a sequence complementary to the Shine-Dalgarno sequence of the gene encoding the polypeptide to the upstream of the Shine-Dalgarno sequence so as to form a secondary structure that makes the attachment of ribosomes impossible may be making mRNA translation impossible or reducing the rate thereof.
Further, 8) the addition of a promoter, which is to be reverse-transcribed, to the 3' end of the open reading frame (ORF) of the gene sequence encoding the polypeptide (reverse transcription engineering, RTE) may be making an antisense nucleotide complementary to the gene transcript encoding the polypeptide to weaken the activity.

As used herein, the term "enhancement" of the polypeptide activity means that the activity of the polypeptide is increased, as compared to the endogenous activity thereof. The enhancement may be used interchangeably with terms such as activation, up-regulation, overexpression, increase, etc. Here, the activation, enhancement, up-regulation, overexpression, and increase may comprise both cases in which an activity not originally possessed is exhibited, or the activity is enhanced, as compared to the endogenous activity or the activity before modification. The "endogenous activity" means the activity of a specific polypeptide originally possessed by a parent strain before the trait is changed or an unmodified microorganism, when the trait is changed by genetic variation due to natural or artificial factors. This may be used interchangeably with "activity before modification". The fact that the activity of a polypeptide is "enhanced", "up-regulated", "overexpressed", or "increased, as compared to the endogenous activity means that the activity of the polypeptide is improved as compared to the activity and/or concentration (expression level) of the specific polypeptide originally possessed by a parent strain before the trait is changed or an unmodified microorganism.

The enhancement may be achieved through the introduction of a foreign polypeptide or the enhancement of the activity of the endogenous polypeptide, and/or concentration (expression level). The enhancement of the activity of the polypeptide may be confirmed by an increase in the degree of activity and the expression level of the corresponding polypeptide or an increase in the amount of the product released from the corresponding polypeptide.

For the enhancement of the activity of the polypeptide, various methods well known in the art may be applied, and the method is not limited as long as the activity of the desired polypeptide may be enhanced as compared to that of the microorganism before modification. Specifically, genetic engineering and/or protein engineering well known to those skilled in the art, which are routine methods of molecular biology, may be used, but the method is not limited thereto (e.g., Sitnicka et al. Functional Analysis of Genes. Advances in Cell Biology. 2010, Vol. 2. 1-16, Sambrook et al. Molecular Cloning 2012, etc.).

Specifically, the enhancement of the polypeptide of the present disclosure may be:
1) increase in the intracellular copy number of the polynucleotide encoding the polypeptide;
2) replacement of a gene expression regulatory region on a chromosome encoding the polypeptide with a sequence having stronger activity;
3) modification of a nucleotide sequence encoding an initiation codon or 5'-UTR region of the gene transcript encoding the polypeptide;
4) modification of the amino acid sequence of the polypeptide to enhance the activity of the polypeptide;
5) modification of the polynucleotide sequence encoding the polypeptide to enhance the activity of the polypeptide (e.g., modification of the polynucleotide sequence of the polypeptide gene to encode the polypeptide that has been modified to enhance the activity of the polypeptide);
6) introduction of a foreign polypeptide exhibiting the activity of the polypeptide or a foreign polynucleotide encoding the same;
7) codon optimization of the polynucleotide encoding the polypeptide;
8) analysis of the tertiary structure of the polypeptide to select and to modify or chemically modify the exposed site; or
9) a combination of two or more selected from 1) to 8), but is not particularly limited thereto.

More specifically, 1) the increase in the intracellular copy number of the polynucleotide encoding the polypeptide may be achieved by introducing, into a host cell, a vector which may replicate and function independently of the host and to which the polynucleotide encoding the corresponding polypeptide is operably linked. Alternatively, the increase may be achieved by introducing one copy or two or more copies of the polynucleotide encoding the corresponding polypeptide into a chromosome of a host cell. The introduction into the chromosome may be performed by introducing, into a host cell, a vector capable of inserting the polynucleotide into a chromosome of the host cell, but is not limited thereto. The vector is as described above.

2) The replacement of a gene expression regulatory region (or expression control sequence) on a chromosome encoding the polypeptide with a sequence exhibiting strong activity may be, for example, occurrence of variation in a sequence due to deletion, insertion, nonconservative or conservative substitution, or a combination thereof, or replacement with a sequence exhibiting stronger activity so that the activity of the expression regulatory region is further enhanced. The expression regulatory region may comprise, but is not particularly limited to, a promoter, an operator sequence, a sequence encoding a ribosome binding site, a sequence controlling the termination of transcription and translation, etc. For example, the replacement may be to replace the original promoter with a strong promoter, but is not limited thereto.

Examples of known strong promoters comprise CJ1 to CJ7 promoters (US 7662943 B2), a lac promoter, a trp promoter, a trc promoter, a tac promoter, a lambda phage PR promoter, a PL promoter, a tet promoter, a gapA promoter, a SPL7 promoter, a SPL13(sm3) promoter (US 10584338 B2), an O2 promoter (US 10273491 B2), a tkt promoter, an yccA promoter, etc., but is not limited thereto.

3) The modification of a nucleotide sequence encoding an initiation codon or 5'-UTR region of the gene transcript encoding the polypeptide may be, for example, substitution with a nucleotide sequence encoding another initiation codon having a higher polypeptide expression rate as compared to an endogenous initiation codon, but is not limited thereto.

4) and 5) The modification of the amino acid sequence or the polynucleotide sequence may be occurrence of variation in the sequence due to deletion, insertion, nonconservative or conservative substitution of the amino acid sequence of the polypeptide or the polynucleotide sequence encoding the polypeptide, or a combination thereof, or replacement with an amino acid sequence or polynucleotide sequence improved to exhibit stronger activity or an amino acid sequence or polynucleotide sequence improved to be more active so that the activity of the polypeptide is enhanced, but is not limited thereto. The replacement may be specifically performed by inserting a polynucleotide into a chromosome by homologous recombination, but is not limited thereto. The vector used here may further comprise a selection marker for the confirmation of chromosome insertion.

6) The introduction of a foreign polynucleotide exhibiting the activity of the polypeptide may be introduction of a foreign polynucleotide into a host cell, the foreign polynucleotide encoding a polypeptide exhibiting activity identical or similar to that of the polypeptide. There is no limitation on its origin or sequence as long as the foreign polynucleotide exhibits activity identical or similar to that of the polypeptide. The method used in the introduction may be performed by appropriately selecting a known transformation method by those skilled in the art. As the introduced polynucleotide is expressed in the host cell, the polypeptide may be produced and the activity thereof may be increased.

7) The codon optimization of the polynucleotide encoding the polypeptide may be codon optimization of an endogenous polynucleotide so as to enhance transcription or translation in a host cell, or codon optimization of a foreign polynucleotide so as to perform optimized transcription and translation in a host cell.

8) The analysis of the tertiary structure of the polypeptide to select and to modify or chemically modify the exposed site may be, for example, to determine a template protein candidate according to the degree of similarity of the sequence by comparing the sequence information of a polypeptide to be analyzed with a database storing the sequence information of known proteins, to confirm the structure based on this, and to modify or chemically modify the exposed site to be modified or chemically modified.

Such enhancement of the polypeptide activity may be an increase in the activity or concentration/expression level of the corresponding polypeptide, based on the activity or concentration of the polypeptide expressed in a wild-type or a microbial strain before being modified, or an increase in the amount of a product produced from the corresponding polypeptide, but is not limited thereto.

The modification of a part or the entirety of the polynucleotide in the microorganism of the present disclosure may be induced by: (a) homologous recombination using a vector for chromosomal insertion in a microorganism or genome editing using engineered nuclease (e.g., CRISPR-Cas9), or and/or (b) the treatment with light, such as ultraviolet light and radiation, and/or chemicals, but is not limited thereto. The method of modifying a part or the entirety of the gene may comprise a method by DNA recombinant technology. For example, a nucleotide sequence or vector comprising a nucleotide sequence homologous to a target gene may be introduced into the microorganism to bring about homologous recombination, resulting in the deletion of a part or the entirety of the gene. The nucleotide sequence or vector to be introduced may comprise a dominant selection marker, but is not limited thereto.

With regard to the microorganism of the present disclosure, the variant, the polynucleotide, and L-ornithine, etc. are as described in other aspects.

Still another aspect of the present disclosure provides a method of producing L-ornithine, the method comprising the step of culturing, in a medium, the microorganism comprising the variant of the present disclosure or the polynucleotide of the present disclosure.

The method of producing L-ornithine of the present disclosure may comprise the step of culturing, in a medium, the microorganism comprising the variant of the present disclosure or the polynucleotide of the present disclosure or the vector of the present disclosure.

As used herein, the term "culturing" refers to growing the microorganism of the present disclosure in appropriately adjusted environmental conditions. The culture procedure of the present disclosure may be performed according to appropriate media or culture conditions known in the art. Such a culture procedure may be easily adjusted according to the selected strain by a person skilled in the art. Specifically, the culturing may be in a batch type, a continuous type, and/or a fed-batch type, but is not limited thereto.

As used herein, the "medium" refers to a mixture comprising, as main ingredients, nutrient materials required for culturing the microorganism of the present disclosure, wherein the medium supplies nutrient materials comprising water essential for survival and growth, growth factors, etc. Specifically, as for the media and other culture conditions used for culturing the microorganism of the present disclosure, any medium that is used for the usual culture of microorganisms may be used without particular limitation. However, the microorganism of the present disclosure may be cultured under aerobic conditions in a common medium comprising appropriate carbon sources, nitrogen sources, phosphorus sources, inorganic compounds, amino acids, and/or vitamins, etc., while controlling the temperature, pH, etc.

Specifically, the culture medium for the microorganism of the genus *Corynebacterium* may be found in the literature ["Manual of Methods for General Bacteriology" by the American Society for Bacteriology (Washington D.C., USA, 1981)].

In the present disclosure, the carbon source may comprise carbohydrates, such as glucose, saccharose, lactose, fructose, sucrose, maltose, etc.; sugar alcohols, such as mannitol, sorbitol, etc.; organic acids, such as pyruvic acid, lactic acid, citric acid, etc.; and amino acids, such as glutamic acid, methionine, lysine, etc. In addition, natural organic nutrient sources may be used, such as starch hydrolysates, molasses, blackstrap molasses, rice bran, cassava, bagasse, and corn steep liquor, and specifically, carbohydrates, such as glucose and sterile pretreated molasses (i.e., molasses converted to reduced sugars) may be used, and appropriate amounts of various other carbon sources may be used without limitation. These carbon sources may be used alone or in a combination of two or more thereof, but are not limited thereto.

As for the nitrogen sources, inorganic nitrogen sources, such as ammonia, ammonium sulfate, ammonium chloride, ammonium acetate, ammonium phosphate, ammonium carbonate, ammonium nitrate, etc.; amino acids, such as glutamic acid, methionine, glutamine, etc.; and organic nitrogen sources, such as peptone, NZ-amine, meat extracts, yeast extracts, malt extracts, corn steep liquor, casein hydrolysates, fish or decomposition products thereof, defatted soybean cake or degradation products thereof, etc. may be used. These nitrogen sources may be used alone or in a combination of two or more thereof, but are not limited thereto.

The phosphorus sources may comprise potassium phosphate monobasic, potassium phosphate dibasic, and sodium-comprising salts corresponding thereto. As for inorganic compounds, sodium chloride, calcium chloride, iron chloride, magnesium sulfate, iron sulfate, manganese sulfate, calcium carbonate, etc. may be used, and in addition, amino acids, vitamins, and/or suitable precursors may be comprised. These constituent ingredients or precursors may be added to the medium in a batch or continuous manner. However, the present disclosure is not limited thereto.

Further, the pH of the medium may be adjusted by adding compounds, such as ammonium hydroxide, potassium hydroxide, ammonia, phosphoric acid, and sulfuric acid, to the medium in an appropriate manner during the culture of the microorganism of the present disclosure. In addition, an anti-foaming agent, such as a fatty acid polyglycol ester, may be used to suppress foam formation during the culture. In addition, oxygen or oxygen-containing gas may be injected into the medium to maintain the aerobic state of the medium, or no gas may be injected, or nitrogen, hydrogen, or carbon dioxide gas may be injected to maintain the anaerobic or non-aerobic state of the medium, but is not limited thereto.

In the culture of the present disclosure, the culture temperature may be maintained at 20°C to 45°C, specifically, at 25°C to 40°C, and the culture may be performed for about 10 hours to about 160 hours, but is not limited thereto.

The L-ornithine produced by the culturing of the present disclosure may be released into the medium or may remain in cells.

The method of producing the L-ornithine of the present disclosure may further comprise the steps of preparing the microorganism of the present disclosure, preparing a medium for culturing the microorganism, or a combination thereof (regardless of the order, in any order), for example, before the culturing step.

The method of producing the L-ornithine of the present disclosure may further comprise the step of recovering the L-ornithine from a medium resulting from the culturing (a medium in which culturing has been performed) or the *Corynebacterium glutamicum* strain. The recovering step may be further comprised after the culturing step.

The recovering may involve collecting the desired L-ornithine by using an appropriate method known in the art according to the method of culturing the microorganism of the present disclosure, for example, a batch, continuous, or fed-batch type culture. For example, centrifugation, filtration, treatment with a crystallized protein precipitating agent (salting-out), extraction, sonication, ultrafiltration, dialysis, various types of chromatography, such as molecular sieve chromatography (gel filtration), adsorption chromatography, ion exchange chromatography, and affinity chromatography, etc., HPLC, and a combination of these methods may be used, and the desired L-ornithine may be recovered from the medium or microorganism by using an appropriate method known in the art.

In addition, the method of producing L-ornithine of the present disclosure may further comprise a purification step. The purification may be performed by using an appropriate method known in the art. In an exemplary embodiment, when the method of producing L-ornithine of the present disclosure comprises both the recovering step and the purification step, the recovering step and the purification step may be performed continuously or discontinuously regardless of the order, or may be performed simultaneously or integrated into one step, but is not limited thereto.

With regard to the method of the present disclosure, the variant, the polynucleotide, the vector, and the strain, etc. are as described in other aspects.

Still another aspect of the present disclosure provides a composition for producing L-ornithine, the composition comprising the variant of the present disclosure; the polynucleotide encoding the variant; the vector comprising the polynucleotide; the microorganism comprising the variant, the polynucleotide encoding the variant, or the vector comprising the polynucleotide; a culture of the microorganism; or a combination of two or more thereof.

The composition of the present disclosure may further comprise any appropriate excipient that is usually used in the composition for producing L-ornithine, and such excipients may comprise, for example, a preserving agent, a wetting agent, a dispersing agent, a suspending agent, a buffering agent, a stabilizing agent, an isotonic agent, etc., but are not limited thereto.

Still another aspect of the present disclosure provides use of the variant of the present disclosure, the polynucleotide encoding the variant, the vector comprising the polynucleotide, or the microorganism comprising the polynucleotide of the present disclosure, in producing L-ornithine.

The carbamoyl phosphate synthetase, the variant, the polynucleotide, the vector, the strain, the medium and the L-ornithine, etc. are as described in other aspects.

Still another aspect of the present disclosure provides use of the carbamoyl phosphate synthetase large subunit (CarB) variant derived from the genus *Corynebacterium,* in producing L-ornithine, wherein the variant comprises a substitution of an amino acid corresponding to position 799 with isoleucine, leucine, or valine; a substitution of an amino acid corresponding to position 918 with leucine or valine; a substitution of an amino acid corresponding to position 1079 with isoleucine, leucine, or valine in the amino acid sequence of SEQ ID NO: 1; or a combination thereof.

### [Mode for Carrying Out the Invention]

Hereinafter, the present disclosure will be described in more detail by way of exemplary embodiments. However, the following exemplary embodiments are only preferred embodiments for illustrating the present disclosure, and thus are not intended to limit the scope of the present disclosure thereto. Meanwhile, technical matters not described in the present specification may be sufficiently understood and easily implemented by those skilled in the technical field of the present disclosure or similar technical fields.

### Example 1: Construction of carB effective mutation-introduced vectors

In order to increase L-ornithine production, CarB variants were prepared, in which amino acids at specific positions (SEQ ID NOS: 1 to 799, 918, or 1079) in an amino acid sequence of CarB were replaced with another amino acid.

First, in order to introduce effective mutations into the carB gene, vectors were constructed to replace glutamic acid at position 799 in the protein sequence of the carB gene with four kinds of amino acids (alanine, isoleucine, leucine, and valine) by considering similar sizes and chemical properties to valine.

To construct a vector for replacement with alanine, the genome of wild-type *C. glutamicum* ATCC 13869 was used as a template, and the homologous recombinant A arm and homologous recombinant B arm were amplified using a primer pair of SEQ ID NOS: 27 and 28 and a primer pair of SEQ ID NOS: 29 and 30, respectively. Similarly, to construct a vector for replacement with isoleucine, homologous recombinant A arm and B arm were amplified using a primer pair of SEQ ID NOS: 27 and 28 and a primer pair of SEQ ID NOS: 31 and 30, respectively. To construct a vector for replacement with leucine, homologous recombinant A arm and B arm were amplified using a primer pair of SEQ ID NOS: 27 and 28 and a primer pair of SEQ ID NOS: 32 and 31, respectively. To construct a vector for replacement with valine, homologous recombinant A arm and B arm were amplified using a primer pair of SEQ ID NOS: 27 and 28 and a primer pair of SEQ ID NOS: 33 and 30, respectively. Thereafter, plasmids were obtained using a plasmid extraction method commonly known, and named pDCM2-carB(E799A), pDCM2-carB(E7991), pDCM2-carB(E799L), pDCM2-carB(E799V).

Vectors for replacing glutamic acid at position 918 in the protein sequence of the carB gene with four kinds of amino acids (alanine, isoleucine, leucine, and valine) were constructed.

To construct a vector for replacement with alanine, the genome of wild-type *C. glutamicum* ATCC 13869 was used as a template, and the homologous recombinant A arm and homologous recombinant B arm were amplified using a primer pair of SEQ ID NOS: 34 and 35 and a primer pair of SEQ ID NOS: 36 and 37, respectively. Similarly, to construct a vector for replacement with isoleucine, homologous recombinant A arm and B arm were amplified using a primer pair of SEQ ID NOS: 34 and 35 and a primer pair of SEQ ID NOS: 38 and 37, respectively. To construct a vector for replacement with leucine, homologous recombinant A arm and B arm were amplified using a primer pair of SEQ ID NOS: 34 and 35 and a primer pair of SEQ ID NOS: 39 and 37, respectively. To construct a vector for replacement with valine, homologous recombinant A arm and B arm were amplified using a primer pair of SEQ ID NOS: 34 and 35 and a primer pair of SEQ ID NOS: 40 and 37, respectively. Thereafter, plasmids were obtained using a plasmid extraction method commonly known, and named in this order: pDCM2-carB(E918A), pDCM2-carB(E9181), pDCM2-carB(E918L), pDCM2-carB(E918V).

Vectors for replacing threonine at position 1079 in the protein sequence of the carB gene with four kinds of amino acids (alanine, isoleucine, leucine, and valine) were constructed.

To construct a vector for replacement with alanine, the genome of wild-type *C. glutamicum* ATCC 13869 was used as a template, and the homologous recombinant A arm and homologous recombinant B arm were amplified using a primer pair of SEQ ID NOS: 41 and 42 and a primer pair of SEQ ID NOS: 43 and 44, respectively. Similarly, to construct a vector for replacement with isoleucine, homologous recombinant A arm and B arm were amplified using a primer pair of SEQ ID NOS: 41 and 42 and a primer pair of SEQ ID NOS: 45 and 44, respectively. To construct a vector for replacement with leucine, homologous recombinant A arm and B arm were amplified using a primer pair of SEQ ID NOS: 41 and 42 and a primer pair of SEQ ID NOS: 46 and 44, respectively. To construct a vector for replacement with valine, homologous recombinant A arm and B arm were amplified using a primer pair of SEQ ID NOS: 41 and 42 and a primer pair of SEQ ID NOS: 47 and 44, respectively. Thereafter, plasmids were obtained using a plasmid extraction method commonly known, and named in this order: pDCM2-carB(T1079A), pDCM2-carB(T10791), pDCM2-carB(T1079L), pDCM2-carB(T1079V).

The sequences of the primers used in Example 1 are as in Table 1 below.

**[Table 1]**

| SEQ ID NO. | Sequence name | Sequence (5'-> 3') |
|---|---|---|
| 27 | P18 | CGGTACCCGGGGATCCTCTTGAGCTCTCCCGC GTC |
| 28 | P19 | GATGTGTTCCATGACGCCC |
| 29 | P20 | GGCGTCATGGAACACATCGCAGAAGCCGGCAT TCACTC |
| 30 | P21 | ATGCCTGCAGGTCGACCGCGGTTAGCCACGGT CAC |
| 31 | P22 | GGCGTCATGGAACACATCATCGAAGCCGGCATT CACTC |
| 32 | P23 | GGCGTCATGGAACACATCCTCGAAGCCGGCATT CACTC |
| 33 | P24 | GGCGTCATGGAACACATCGTCGAAGCCGGCAT TCACTC |
| 34 | P25 | CGGTACCCGGGGATCCGGAGATTGTCTACGATG AG |
| 35 | P26 | CTTCACAGCGATTGGAGCG |
| 36 | P27 | GCTCCAATCGCTGTGAAGGCAGCAGTGTTGCC GTTCAAC |
| 37 | P28 | ATGCCTGCAGGTCGACCTGACAACGCCCTCAC GCAG |
| 38 | P29 | GCTCCAATCGCTGTGAAGATCGCAGTGTTGCCG TTCAAC |
| 39 | P30 | GCTCCAATCGCTGTGAAGCTCGCAGTGTTGCC GTTCAAC |
| 40 | P31 | GCTCCAATCGCTGTGAAGGTCGCAGTGTTGCC GTTCAAC |
| 41 | P32 | CGGTACCCGGGGATCCCAAGGATCTCCAAGAT GAG |
| 42 | P33 | GATCAGTGGAACACCCACG |
| 43 | P34 | GTGGGTGTTCCACTGATCGCAACTGTCCAGGG TGTCAC |
| 44 | P35 | ATGCCTGCAGGTCGACCTCCCCTGCCGTGTTG CTC |
| 45 | P36 | GTGGGTGTTCCACTGATCATCACTGTCCAGGGT GTCAC |
| 46 | P37 | GTGGGTGTTCCACTGATCCTCACTGTCCAGGGT GTCAC |
| 47 | P38 | GTGGGTGTTCCACTGATCGTCACTGTCCAGGGT GTCAC |

### Example 2: Preparation of microorganism producing L-ornithine

To prepare a microorganism producing L-ornithine, a vector was constructed, in which the vector replaces serine at position 55 of a protein sequence of ArgF (ANU33618.1, SEQ ID NO: 48) with a stop codon. The genome of wild-type C. *glutamicum* ATCC1 3869 was used as a template, and homologous recombinant A arm and homologous recombinant B arm were amplified using a primer pair of SEQ ID NOS: 49 and 50 and a primer pair of SEQ ID NOS: 51 and 52, respectively. Thereafter, a plasmid was obtained in the same manner as above, and named pDCM2-argF(S55*).

To prepare a microorganism in which the L-ornithine-producing ability was further improved, a vector was constructed, in which the vector replaces glutamic acid at position 47 of a protein sequence of ArgR (ANU33619.1, SEQ ID NO: 53) with a stop codon. The genome of wild-type *C. glutamicumATCC1* 3869 was used as a template, and homologous recombinant A arm and homologous recombinant B arm were amplified using a primer pair of SEQ ID NOS: 54 and 55 and a primer pair of SEQ ID NOS: 56 and 57, respectively. Thereafter, a plasmid was obtained in the same manner as above, and named pDCM2-argR(E47*).

The sequences of the primers used in Example 2 are as in Table 2 below.

**[Table 2]**

| SEQ ID NO. | Sequence name | Sequence (5'-> 3') |
|---|---|---|
| 49 | P4 | CGGTACCCGGGGATCCTGACCCCAGGCAAG CACGG |
| 50 | P5 | GAAGCGAGTACGAGTTTAAGTCTTATC |
| 51 | P6 | AAACTCGTACTCGCTTCTCC |
| 52 | P7 | ATGCCTGCAGGTCGACCGGCGCCGGCAACC TCGTC |
| 54 | P8 | CGGTACCCGGGGATCCCTCGTGCGGAATTC GTGGAG |
| 55 | P9 | ATCCAGCAGCAATTCAGACA |
| 56 | P10 | CTGAATTGCTGCTGGATTAAGGCATCGATATC ACCCA |
| 57 | P11 | ATGCCTGCAGGTCGACCCTTCATTTTAAGTTC CTTG |

The wild-type C. *glutamicum* ATCC 13869 was transformed with the pDCM2-argF(S55*) vector constructed above by electroporation (Appl. Microbiol.Biotechnol. (1999) 52:541-545), and then a second crossover process was performed to obtain a microorganism, in which a nucleotide sequence at position 164 of argF was substituted from cytosine (c) to adenine (a) and thus a protein sequence at position 55 was substituted with a stop codon. PCR and sequencing analysis were performed using a primer pair of SEQ ID NOS: 49 and 52 which are capable of amplifying the adjacent region comprising the position where the corresponding gene was inserted, and the corresponding genetic manipulation was confirmed. The microorganism thus obtained was named C. gl::argF*.

To prepare a microorganism in which ornithine production was further improved in C. gl::argF*, the pDCM2-argR(E47*) vector was used to obtain a microorganism in the same manner as above. PCR and sequencing analysis were performed using a primer pair of SEQ ID NOS: 54 and 57 which are capable of amplifying the adjacent region comprising the position where the corresponding gene was inserted, and the corresponding genetic manipulation was confirmed. The microorganism thus obtained was named C. gl::argF*_argR*.

### Example 3: Preparation of carB mutant strain based on L-ornithine-producing strain

To prepare mutant strains for glutamic acid at position 799 of the carB protein sequence, based on the L-ornithine-producing strain, C. gl::argF*_argR* prepared in Example 2 was transformed with pDCM2-carB(E799A), pDCM2-carB(E7991), pDCM2-carB(E799L), and pDCM2-carB(E799V) vectors constructed in Example 1 by electroporation, respectively and then a second crossover process was performed to obtain microorganisms, each in which glutamic acid was substituted with alanine, isoleucine, leucine, or valine. PCR and sequencing analysis were performed using a primer pair of SEQ ID NOS: 27 and 30 which are capable of amplifying the adjacent region comprising the position where the corresponding gene was inserted, and the corresponding genetic manipulation was confirmed. The microorganisms thus obtained were named C. gl::argF*_argR*_carB(E799A), C. gl::argF*_argR*_carB(E799I), C. gl::argF*_argR*_carB(E799L), C. gl::argF*_argR*_carB(E799V), respectively.

To prepare mutant strains for glutamic acid at position 918 of the carB protein sequence, based on the L-ornithine-producing strain, C. gl::argF*_argR* prepared in Example 2 was transformed with pDCM2-carB(E918A), pDCM2-carB(E9181), pDCM2-carB(E918L), and pDCM2-carB(E918V) vectors constructed in Example 5 by electroporation, respectively and then a second crossover process was performed to obtain microorganisms, each in which glutamic acid was substituted with alanine, isoleucine, leucine, or valine. PCR and sequencing analysis were performed using a primer pair of SEQ ID NOS: 34 and 37 which are capable of amplifying the adjacent region comprising the position where the corresponding gene was inserted, and the corresponding genetic manipulation was confirmed. The microorganisms thus obtained were named C. gl::argF*_argR*_carB(E918A), C. gl::argF*_argR*_carB(E918I), C. gl::argF*_argR*_carB(E918L), C. gl::argF*_argR*_carB(E918V), respectively.

To prepare mutant strains for threonine at position 1079 of the carB protein sequence, based on the L-ornithine-producing strain, C. gl::argF*_argR* prepared in Example 2 was transformed with pDCM2-carB(T1079A), pDCM2-carB(T10791), pDCM2-carB(T1079L), and pDCM2-carB(T1079V) vectors constructed in Example 5 by electroporation, respectively and then a second crossover process was performed to obtain microorganisms, each in which glutamic acid was substituted with alanine, isoleucine, leucine, or valine. PCR and sequencing analysis were performed using a primer pair of SEQ ID NOS: 41 and 44 which are capable of amplifying the adjacent region comprising the position where the corresponding gene was inserted, and the corresponding genetic manipulation was confirmed. The microorganisms thus obtained were named C. gl::argF*_argR*_carB(T1079A), C. gl::argF*_argR*_carB(T1079I), C. gl::argF*_argR*_carB(T1079L), C. gl::argF*_argR*_carB(T1079V), respectively.

### Example 4: Comparison of L-ornithine-producing ability between carB mutant strains

C. gl::argF*_argR*_carB(E799A), C. gl::argF*_argR*_carB(E799I), C. gl::argF*_argR*_carB(E799L), C. gl::argF*_argR*_carB(E799V), C. gl::argF*_argR*_carB(E918A), C. gl::argF*_argR*_carB(E918I), C. gl::argF*_argR*_carB(E918L), C. gl::argF*_argR*_carB(E918V), C. gl::argF*_argR*_carB(T1079A), C. gl::argF*_argR*_carB(T1079I), C. gl::argF*_argR*_carB(T1079L), C. gl::argF*_argR*_carB(T1079V) microorganisms prepared in Example 3, and control microorganisms, wild-type *C. glutamicum ATCC* 13869 and C. gl::argF*_argR* microorganism were cultured using a production medium below, and the cell mass and L-ornithine-producing ability were compared.

First, each microorganism was inoculated into a 250 ml corner-baffled flask comprising 25 ml of a seed medium, and cultured at 30°C for 16 hours with shaking at 200 rpm. 1 ml of the seed culture was inoculated into a 250 ml corner-baffled flask comprising 24 ml of a production medium, and cultured at 33°C for 40 hours with shaking at 200 rpm.

### <Seed medium (pH 7.0)>

20 g of glucose, 10 g of peptone, 5 g of yeast extract, 1.5 g of urea, 4 g of KH₂PO₄, 8 g of K₂HPO₄, 0.5 g of MgSO₄·7H₂O, 0.1 mg of biotin, 1 mg of thiamine HCl, 22 mg of calcium pantothenate, 2 mg of nicotinamide (based on 1 liter of distilled water)

### <Production medium (pH 7.0)>

50 g of raw sugar, 25 g of (NH₄)₂SO₄, 1 g of yeast extract, 0.55 g of KH₂PO₄, 0.6 g of MgSO₄·7H₂O, 0.2 g of L-arginine, 0.9 mg of biotin, 4.5 mg of thiamine HCl, 4.5 mg of calcium pantothenate, 30 mg of nicotinamide, 9 mg of MnSO₄, 9 mg of FeSO₄, 0.45 mg of ZnSO₄, 0.45 mg of CuSO₄, 30 g of CaCOs (based on 1 liter of distilled water)

The above experiment was repeated three times, and the culture results (average value) are shown in Table 3 below. The L-ornithine yield was calculated by ornithine concentration/sugar consumption, and the concentration increase rate, L-ornithine yield was calculated by ornithine concentration/sugar consumption, the concentration increase rate was calculated by (concentration of L-ornithine produced by experimental group-concentration of L-ornithine produced by the control group (wild-type *C. glutamicum* ATCC 13869 strain))/concentration of L-ornithine produced by the control group (wild-type *C. glutamicum* ATCC 13869 strain).

**[Table 3]**

| | Strain | OD₅₆₂ | Sugar consumptio n (g/L) | L-ornithine concentration (g/L) | L-ornithine yield (%) | Increase rate of L-ornithine concentration (%) |
|---|---|---|---|---|---|---|
| Control microorganis m | Wild-type C. *glutamicum* ATCC 13869 | 95.8 | 50.0 | 0.0 | 0.0 | - |
| | C. gl::argF*_argR * | 41.4 | 50.0 | 17.4 | 34.8 | 17 |
| Microorganis m with substitution of | C. gl::argF*_argR *_carB(E799A) | 44.2 | 50.0 | 16.3 | 32.6 | 16 |
| glutamic acid at position 799 | C. gl::argF*_argR *_carB(E799I) | 42.7 | 50.0 | 18.7 | 37.4 | 19 |
| | C. gl::argF*_argR *_carB(E799L) | 43.6 | 50.0 | 20.1 | 40.2 | 20 |
| | C. gl::argF*_argR *_carB(E799V) | 43.2 | 50.0 | 19.2 | 38.4 | 19 |
| Microorganis m with substitution of glutamic acid at position 918 | C. gl::argF*_argR *_carB(E918A) | 41.2 | 50.0 | 14.5 | 29.0 | 15 |
| | C. gl::argF*_argR *_carB(E918I) | 43.9 | 50.0 | 17.3 | 34.6 | 17 |
| | C. gl::argF*_argR *_carB(E918L) | 45.1 | 50.0 | 17.9 | 35.8 | 18 |
| | C. gl::argF*_argR *_carB(E918V) | 43.6 | 50.0 | 17.8 | 35.6 | 18 |
| Microorganis m with substitution of threonine at position 1079 | C. gl::argF*_argR *_carB(T1079 A) | 43.1 | 50.0 | 16.9 | 33.8 | 17 |
| | C. gl::argF*_argR *_carB(T1079I ) | 44.0 | 50.0 | 18.2 | 36.4 | 18 |
| | C. gl::argF*_argR *_carB(T1079 L) | 43.0 | 50.0 | 18.7 | 37.4 | 19 |
| | C. gl::argF*_argR *_carB(T1079 V) | 44.8 | 50.0 | 19.6 | 39.2 | 20 |

As shown in Table 3, C. gl::argF*_argR*_carB(E799I), C. gl::argF*_argR*_carB(E799L), C. gl::argF*_argR*_carB(E799V), C. gl::argF*_argR*_carB(E918L), C. gl::argF*_argR*_carB(E918V), C. gl::argF*_argR*_carB(T1079I), C. gl::argF*_argR*_carB(T1079L), C. gl::argF*_argR*_carB(T1079V) showed increased production ability and yield, as compared to the control microorganism C. gl::argF*_argR*.

Based on the above description, a person skilled in the art to which the present disclosure pertains can understand that the present disclosure may be embodied in other specific forms without departing from the technical spirit or essential characteristics thereof. In this regard, the embodiments described above should be understood to be illustrative rather than restrictive in every respect. The scope of the present disclosure should be construed as the meaning and scope of the appended claims rather than the detailed description, and all changes or variations derived from the equivalent concepts falling within the scope of the present disclosure.

## Claims

1. A carbamoyl phosphate synthetase large subunit variant derived from the genus *Corynebacterium,* the variant comprising a substitution of an amino acid corresponding to position 799 with isoleucine, leucine, or valine; a substitution of an amino acid corresponding to position 918 with leucine or valine; a substitution of an amino acid corresponding to position 1079 with isoleucine, leucine, or valine in an amino acid sequence of SEQ ID NO: 1; or a combination thereof.

2. A polynucleotide encoding the variant of claim 1.

3. The carbamoyl phosphate synthetase large subunit variant of claim 1, wherein the carbamoyl phosphate synthetase large subunit variant has 80% or more sequence identity to SEQ ID NO: 1.

4. A microorganism of the genus *Corynebacterium* comprising a carbamoyl phosphate synthetase large subunit variant or a polynucleotide encoding the variant, wherein the variant comprises a substitution of an amino acid corresponding to position 799 with isoleucine, leucine, or valine; a substitution of an amino acid corresponding to position 918 with leucine or valine; a substitution of an amino acid corresponding to position 1079 with isoleucine, leucine, or valine in an amino acid sequence of SEQ ID NO: 1; or a combination thereof.

5. The microorganism of claim 4, wherein the microorganism has L-ornithine-producing ability.

6. The microorganism of claim 4, wherein activity of ornithine carbamoyltransferase (ArgF) is further weakened.

7. The microorganism of claim 4, wherein activity of arginine repressor (ArgR) is further weakened.

8. The microorganism of claim 4, wherein the microorganism of the genus *Corynebacterium* is *Corynebacterium glutamicum.*

9. A method of producing L-ornithine, the method comprising the step of culturing, in a medium, a microorganism of the genus *Corynebacterium* comprising a carbamoyl phosphate synthetase large subunit variant or a polynucleotide encoding the variant, wherein the variant comprises a substitution of an amino acid corresponding to position 799 with isoleucine, leucine, or valine; a substitution of an amino acid corresponding to position 918 with leucine or valine; a substitution of an amino acid corresponding to position 1079 with isoleucine, leucine, or valine in an amino acid sequence of SEQ ID NO: 1; or a combination thereof.

10. The method of claim 9, wherein activity of ornithine carbamoyltransferase (ArgF) is further weakened.

11. The method of claim 9, wherein activity of arginine repressor (ArgR) is further weakened.

12. A composition for producing L-ornithine, the composition comprising a carbamoyl phosphate synthetase large subunit variant derived from the genus *Corynebacterium,* the variant comprising a substitution of an amino acid corresponding to position 799 with isoleucine, leucine, or valine; a substitution of an amino acid corresponding to position 918 with leucine or valine; a substitution of an amino acid corresponding to position 1079 with isoleucine, leucine, or valine in an amino acid sequence of SEQ ID NO: 1; or a combination thereof; a polynucleotide encoding the variant; a vector comprising the polynucleotide; a microorganism comprising the variant, the polynucleotide encoding the variant, or the vector comprising the polynucleotide; a culture of the microorganism, or a combination of two or more thereof.

13. Use of a microorganism of the genus *Corynebacterium* in producing L-ornithine, the microorganism comprising a carbamoyl phosphate synthetase large subunit variant comprising a substitution of an amino acid corresponding to position 799 with isoleucine, leucine, or valine; a substitution of an amino acid corresponding to position 918 with leucine or valine; a substitution of an amino acid corresponding to position 1079 with isoleucine, leucine, or valine in an amino acid sequence of SEQ ID NO: 1; or a combination thereof; or a polynucleotide encoding the variant.
